# EUROPEAN PATENT SPECIFICATION

(11) **EP 4 496 522 B1**
(45) Date of publication and mention of the grant of the patent: **15.07.2026**
(21) Application number: 23719939.3
(22) Date of filing: 20.03.2023
(51) Int. Cl.: A61B 17/34, A61B 17/32, A61F 2/04, A61B 17/22

(54) **TISSUE MANIPULATING SYSTEMS**
GEWEBEMANIPULATIONSSYSTEME
SYSTÈMES DE MANIPULATION DE TISSU

(30) Priority: 23.03.2022 US 202263322662 P; 10.06.2022 US 202263350929 P
(43) Date of publication of application: 29.01.2025
(60) Divisional application: 26183037.6
(73) Proprietor: Teleflex Life Sciences LLC, Wilmington, DE 19808 (US)
(72) Inventor: DUBOIS, Brian R., Pleasanton, California 94588 (US); JOHNSTON, Andrew L., Pleasanton, California 94588 (US); WELCH, Jacqueline Nerney, Pleasanton, California 94588 (US); LAMSON, Theodore C., Pleasanton, California 94588 (US); CATANESE III, Joseph, Pleasanton, California 94588 (US)
(74) Representative: Mewburn Ellis LLP
(86) International application number: PCT/US2023/015645
(87) International publication number: WO 2023/183238

(56) References cited:
- WO-A1-2021/250588
- KR-B1- 101 534 820
- US-A- 5 817 127
- US-A1- 2013 253 574
- US-A1- 2013 261 540
- US-A1- 2016 000 455
- US-A1- 2019 307 548
- US-A1- 2022 061 834

## Description

### CLAIM OF PRIORITY

Benefit of priority is hereby claimed to U.S. Provisional Patent Application Serial Number 63/322,662, entitled "TISSUE CUTTING SYSTEM AND METHODS OF USE" and filed on March 23, 2022, and to U.S. Provisional Patent Application Serial Number 63/350,929, entitled "TISSUE MANIPULATING SYSTEM AND METHODS OF USE" and filed June 10, 2022.

### TECHNICAL FIELD

The present disclosure relates generally to medical devices and methods, and more particularly to systems and associated methods for manipulating, cutting, and/or retracting tissues and anatomical or other structures within the body of human or animal subjects for the purpose of treating diseases or disorders, such as prostate conditions.

### BACKGROUND

One example of a condition where it is desirable to manipulate or retract tissue is Benign Prostatic Hyperplasia (BPH). BPH is one of the most common medical conditions that affect men, especially elderly men. It has been reported that, in the United States, more than half of all men have histopathologic evidence of BPH by age 60 and, by age 85, approximately 9 out of 10 men suffer from the condition. Moreover, the incidence and prevalence of BPH are expected to increase as the average age of the population in developed countries increases.

Certain treatments for BPH involve gaining access to the urinary tract. New devices and methods have been developed for various procedures to manipulate or retract prostatic tissue in a discrete procedure or in combination with treating BPH. Some procedures remove tissue, and other procedures modify tissue.

One example of a surgical procedure for treating BPH symptoms is Transurethral Incision of the Prostate (TUIP). In this procedure, the resistance to urine flow is reduced by making one or more incisions in the prostate gland in the region where the urethra meets the urinary bladder, known as the bladder neck. This procedure is performed under general or spinal anesthesia. In this procedure, one or more incisions are made in the muscle of the bladder neck. The incisions in most cases are deep enough to cut the surrounding prostate gland tissue including the prostatic capsule. This releases any compression on the bladder neck and causes the bladder neck to spring apart. The incisions can be made using a resectoscope, a laser beam, or other methods.

Another method of interest uses a drug coated balloon to enlarge the urinary tract in the area of the prostatic urethra. The drug coated balloon is inflated and causes damage to at least the encroaching tissue and perhaps to the underlying tissue. In a typical balloon procedure, tissue of the entire inner circumference of the prostatic urethra is damaged by the expanding balloon. The drug coating on the balloon helps mitigate an overproliferative healing response such that the prostatic urethra is left less obstructed than before the procedure. However, this procedure may cause more tissue damage than is necessary to treat the symptoms of BPH.

US 5 817 127 A discloses a urological instrument that combines functions of a mechanical urethral dilator with functions of a urethrotome. US 2013/261540 A1 discloses a needleless or high-pressure injection system for injecting treatment or therapeutic fluid to tissue of the lower urinary tract, such as the prostate or bladder.

### BRIEF SUMMARY OF THE INVENTION

The present inventors recognize that there remains a need to treat the minimum amount of tissue necessary to relieve the symptoms of BPH in a minimally invasive and low-risk procedure. The present disclosure addresses these needs.

The invention is defined by claim 1. Further embodiments of the invention are defined by the dependent claims.

Aspects of the present disclosure are directed towards systems and methods for treating, manipulating, and stabilizing tissue within a prostatic urethra to improve symptoms of BPH. Certain embodiments involve treating tissue within a prostatic urethra by cutting the tissue and delivering a therapeutic substance.

Embodiments of this disclosure are directed to a system for treating tissue in the prostatic urethra including a handle body having a cutter actuator and an inflation port. Embodiments of a system may also include an elongate member coupled to a distal end of the handle body and to a cutter body. An expandable member may be coupled to a distal end of the elongate member. The expandable member may be configured to stabilize the cutter body against target tissue when the expandable member is expanded. The cutter may be configured to move from a first position within the cutter body to a second position protruding from a cutter slot and to a third position again within the cutter body. An injection port on the handle body in fluid communication with the cutter slot may be configured to supply a therapeutic substance through the cutter slot to target tissue.

Embodiments of this disclosure are directed to systems for manipulating tissue in the prostatic urethra including a handle body having a deployment actuator and an inflation port. Embodiments of a system may include an elongate member coupled to a distal end of the handle body. The system may include a delivery body and an expandable member coupled to a distal end of the elongate member. The expandable member may be configured to stabilize the delivery body against target tissue when the expandable member is expanded. A tissue manipulation structure may be configured to move from a first position within the delivery body to a second position at least partially within prostatic tissue. The movement of the tissue manipulation structure may be controlled via the deployment actuator.

Other features and advantages of embodiments of the present disclosure and invention will become apparent from the following description, taken in conjunction with the accompanying drawings, which illustrate, by way of example, certain principles of the invention. These illustrative aspects are mentioned not to limit or define the disclosure, but to provide examples to aid understanding thereof.

### BRIEF DESCRIPTION OF THE SEVERAL VIEWS OF THE DRAWINGS

Features, aspects, and advantages of the present disclosure are better understood when the following Detailed Description is read with reference to the accompanying drawings. In the drawings, like numerals can be used to describe similar features and components throughout several drawings. The drawings illustrate generally, by way of example, but not by way of limitation, various device and method aspects discussed in this patent document.
FIG. 1 is a cross-sectional view illustrating anatomy surrounding a prostate in a human subject.
FIG. 2 is a view of tissue from within the prostatic urethra.
FIG. 3 is a perspective view of a system according to aspects of the present disclosure.
FIG. 4A is a perspective view of a distal portion of the system according to aspects of the present disclosure.
FIG. 4B is a perspective view of a distal portion of the system according to aspects of the present disclosure.
FIG. 5 is a perspective view of a distal portion of the system according to aspects of the present disclosure.
FIG. 6A is a side view with partial transparency of a distal portion of the system according to aspects of the present disclosure.
FIG. 6B is a side view with partial transparency of a distal portion of the system according to aspects of the present disclosure.
FIG. 6C is a side view with partial transparency of a distal portion of the system according to aspects of the present disclosure.
FIG. 6D is a side view with partial transparency of a distal portion of the system according to aspects of the present disclosure.
FIG. 6E is a perspective view of a distal portion of the system according to aspects of the present disclosure.
FIG. 7A is a plan view of a proximal portion of the system according to aspects of the present disclosure.
FIG. 7B is a plan view of a proximal portion of the system according to aspects of the present disclosure.
FIG. 8A is a perspective view of a proximal portion of the system according to aspects of the present disclosure.
FIG. 8B is a perspective view of a proximal portion of the system according to aspects of the present disclosure.
FIG. 9A is a flowchart illustrating steps in a method for using the system according to aspects of the present disclosure.
FIG. 9B is a flowchart illustrating steps in another method for using the system according to aspects of the present disclosure.
FIG. 10 is a perspective view of another system according to aspects of the present disclosure.
FIG. 11 is a perspective view of a system according to aspects of the present disclosure.
FIG. 12 is a perspective view of a tissue manipulation structure according to aspects of the present disclosure.
FIG. 13 is a perspective view of a distal portion of a system according to aspects of the present disclosure.
FIG. 14 is a perspective view of a distal portion of a system according to aspects of the present disclosure.
FIG. 15 is a side view with partial transparency of a distal portion of a system according to aspects of the present disclosure.
FIG. 16A is a flowchart illustrating steps in a method for using a system according to aspects of the present disclosure.
FIG. 16B is a flowchart illustrating steps in another method for using a system according to aspects of the present disclosure.

### DETAILED DESCRIPTION OF THE INVENTION

Numerous specific details are set forth herein to provide a thorough understanding of the claimed subject matter. However, those skilled in the art will understand that the claimed subject matter may be practiced without these specific details. In other instances, methods, apparatuses, or systems that would be known by one of ordinary skill have not been described in detail so as not to obscure claimed subject matter.

The use of "adapted to" or "configured to" herein is meant as open and inclusive language that does not foreclose devices adapted to or configured to perform additional tasks or steps. The use of "proximal" and "distal" herein refers to relative positions with respect to a user of the elongate, minimally invasive devices, where "proximal" means relatively towards the user and "distal" means relatively away from the user. Headings, lists, and numbering included herein are for ease of explanation only and are not meant to be limiting.

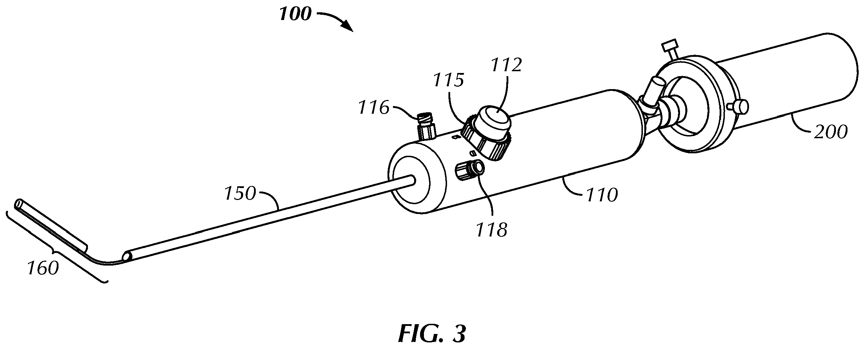

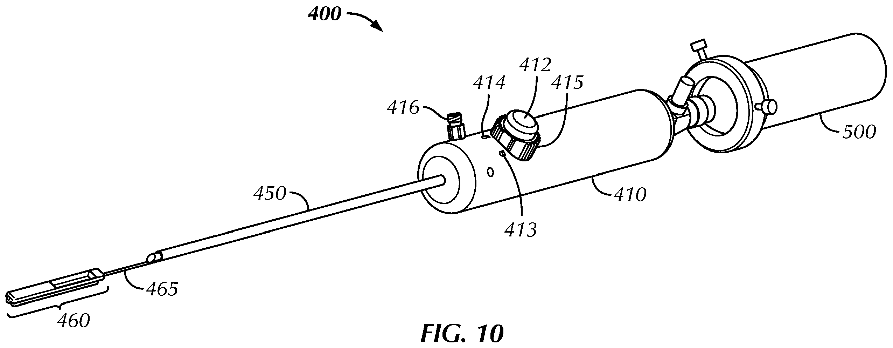

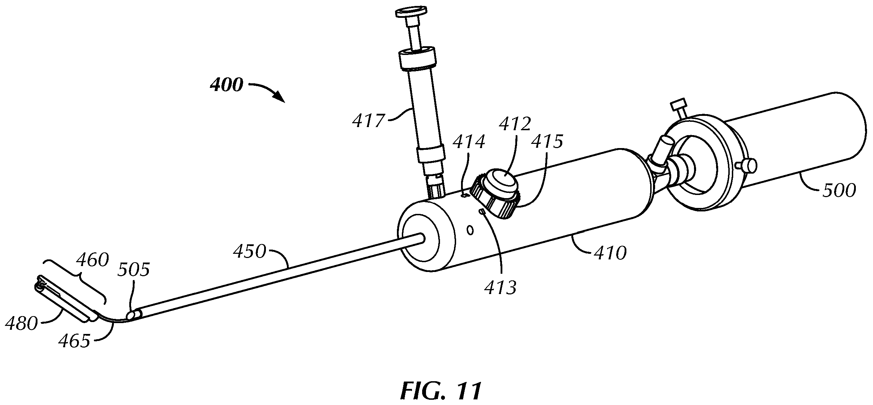

As used herein, the term "manipulating" may refer to "cutting," "retracting," and/or "stabilizing" tissue. In other examples, "manipulating" may refer to a distinct activity relative to terms like "cutting." For example, cutting tissue may refer to slicing the tissue using the system 100 depicted in FIG. 3, whereas manipulating tissue may refer to creating and/or stabilizing an opening in a commissure using the system depicted in FIGS. 10 and/or 11.

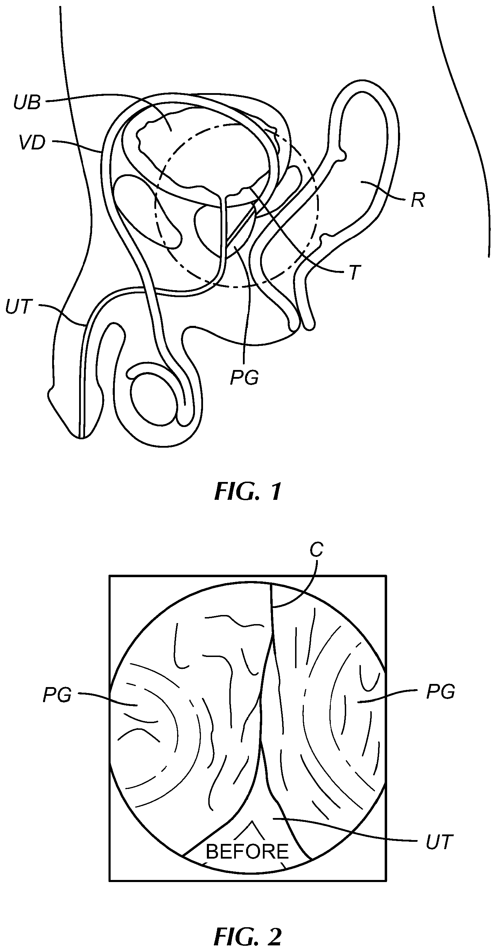

FIG. 1 is a cross-sectional view illustrating anatomy surrounding a prostate in a human subject. The prostate gland PG is a walnut-sized muscular gland located adjacent the urinary bladder UB. The urethra UT runs through the prostate gland PG. The prostate gland PG secretes fluid that protects and nourishes sperm. The prostate gland PG also contracts during ejaculation of sperm to expel semen and to provide a valve to keep urine out of the semen. The urinary bladder UB holds urine. The vas deferentia VD define ducts through which semen is carried and the seminal vesicles secrete seminal fluid. The rectum R is the end segment of the large intestine and through which waste is dispelled. The urethra UT carries both urine and semen out of the body. Thus, the urethra is connected to the urinary bladder UB and provides a passageway to the vas deferentia VD and seminal vesicles. Further, the trigone T is a smooth triangular end of the bladder. It is sensitive to expansion and signals the brain when the urinary bladder UB is full. The verumontanum is a crest in the wall of the urethra UT where the seminal ducts enter. The prostatic urethra is the section of the urethra UT that extends through the prostate.

The prostate gland can be described by referring to its lobes. Whereas zone classification is typically used in pathology, the lobe classification is more often used in anatomy. The central zone of a prostate gland PG is about 25% of a normal prostate and this zone surrounds the ejaculating ducts. There is some prevalence of benign prostatic hyperplasia in the transition zone. The fibromuscular zone is usually devoid of glandular components and as its name suggests, is composed of only muscle and fibrous tissue. The transitional zone generally overlays the proximal urethra and is the region of the gland that grows throughout life. Also, this lobe is often associated with the condition of benign prostatic enlargement. Finally, the peripheral zone is the sub-capsular portion of the posterior aspect of the prostate gland that surrounds the distal urethra.

The lobe characterization is different from the zone characterization, but there is some overlap. The anterior lobe is devoid of glandular tissue and is completely formed of fibromuscular tissue. This lobe thus roughly corresponds to the anterior portion of the transitional zone. The posterior lobe roughly corresponds to the peripheral zone and can be palpated through the rectum during a digital rectal exam. The posterior lobe is the site of 70-80% of prostatic cancers. The lateral lobe is the main mass of the prostate and is separated by the urethra. It has been described as spanning all zones. Lastly, the median lobe roughly corresponds to part of the central zone. It varies greatly in size and in some cases is devoid of glandular tissue.

A commissure is present where the lobes abut each other near the anterior, medial aspect of the urethra. In some aspects of the disclosure, the system and method are directed to cutting, manipulating, and/or stabilizing tissue at a commissure. Embodiments may further involve applying a therapeutic substance to the commissure, for instance at the site of a cut. The cut, manipulated, and/or stabilized tissue at the commissure can provide an opening in an otherwise restricted prostatic urethra to enable the fluid pressure from the bladder to allow urine flow through the prostatic urethra. The estimated length of a commissure in various examples may range from about 10 mm to about 50 mm, with a typical length range of 25 ± 5 mm. Commissures of various lengths may be manipulated and treated in accordance with examples described herein.

FIG. 2 is a view of tissue from within the prostatic urethra. FIG. 2 shows two lateral lobes of the prostate gland PG abutting each other to define a commissure C. The prostatic section of the urethra UT is shown in FIG. 2 as significantly reduced in cross-sectional area because of impingement by the prostatic gland. Thus, FIG. 2 illustrates a view of prostatic tissue in need of cutting, manipulation and/or stabilization by the systems and methods disclosed herein.

FIG. 3 is a perspective view of a system according to aspects of the present disclosure. The system 100 is illustrated as coupled to endoscope body 200 at the proximal end of the handle body 110 of the system 100. The handle body 110 includes an actuator 112 operatively connected to the distal section 160 of the system 100. Specifically, the actuator 112 can be operatively connected to a cutter in the distal section 160 of the system 100, and may thus be referred to as the "cutter actuator" in various embodiments. The handle body 110 in the example shown also includes a lock 115 engaged with the actuator 112 to lock or unlock the actuator 112. The lock 115 can engage the actuator 112 to lock it in the sense that the lock 115 prevents the actuator 112 from being operated when in a locked position, thereby preventing movement of the cutter described herein. Conversely, the lock 115 can disengage the actuator 112 to unlock it and allow the actuator to be operated from an unlocked position.

The handle body 110 in the example shown in FIG. 3 includes an inflation port 116 and an injection port 118. Both the inflation port 116 and the injection port 118 are configured to transport fluid to the distal section 160 of the system. The inflation port 116 is configured to transport inflation fluid through an inflation lumen within the handle body 110 and within an elongate member 150 of the system to the distal section 160 of the system 100. Similarly, the injection port 118 is configured to transport injection fluid through an injection lumen within the handle body 110 and within the elongate member 150 of the system to the distal section 160 of the system 100. In some examples of the system 100, the injection lumen is also the lumen through which a cutter wire is connected between the actuator 112 and the distal section 160.

The elongate member 150 connects the handle body 110 with the distal section 160. The elongate member 150 may include an endoscope lumen for the elongated portion of an endoscope configured for use within a patient's body. The elongate member 150 may also include the inflation lumen and injection lumen previously described. In some examples of the system, the elongate member 150 includes a cutter wire within the injection lumen, and in other examples the cutter wire occupies space within the elongate member 150 but separate from the injection lumen. The elongate member 150 may be rigid, semi-rigid, or semi-flexible. The elongate member 150 is configured to be insertable within the urethra of a patient and is composed of materials suitable for medical use.

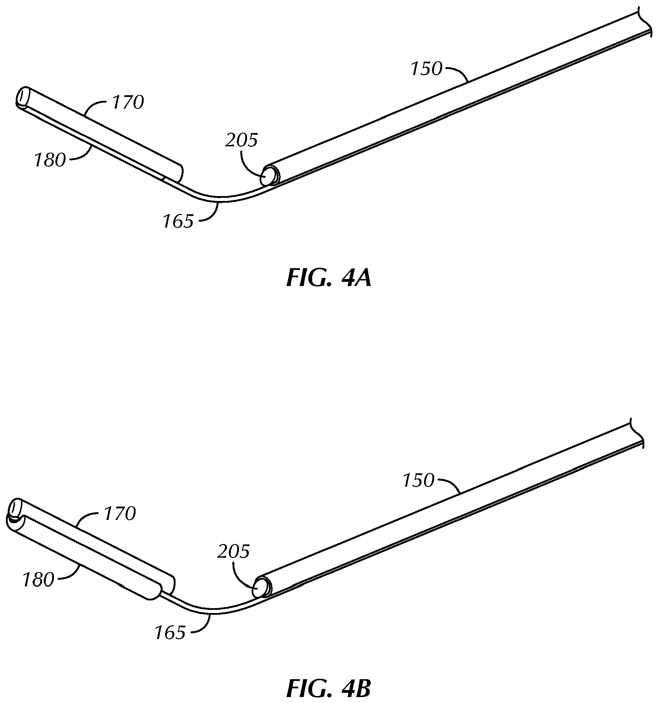

FIG. 4A and FIG. 4B are each a perspective view of a distal portion of the system 100 according to aspects of the present disclosure. In the illustrated example, the elongate member 150 of the system is connected via a curved portion or articulation 165 (or "articulated section") to a cutter body 170 and an expandable member 180. The endoscope distal end 205 can be seen at the distal end of the elongate member 150. In this non-limiting example, the endoscope distal end 205 is a 30-degree cystoscope. Additional endoscopes may be used.

Beyond the distal end of the elongate member 150 is the articulation 165, which may be more flexible than the elongate member 150. The articulation 165 may be configured to curve at a pre-formed angle with respect to the long axis of the elongate member 150. The pre-formed curve in the articulation 165 may be configured to generally match the typical anatomical curvature of the urethra near the prostate and bladder neck. The articulation 165 may be flexible to enable the articulation 165 to conform to the curvature of the urethra and position the distal section 160 in the prostatic urethra without puncturing the urethral wall. The articulation 165 presents the cutter body 170 within easy view of the 30-degree cystoscope. The articulation 165 can be formed from plastics, shape memory materials, or other flexible to semi-flexible materials suitable for medical use. The flexibility of the articulation 165 allows for comparatively easier navigation of the urethra as compared to an entirely rigid device in addition to presenting the cutter body 170 at a desirable angle within the anatomy with respect to the long axis of the elongate member 150. The articulation 165 may include the inflation lumen, the injection lumen, and the cutter wire.

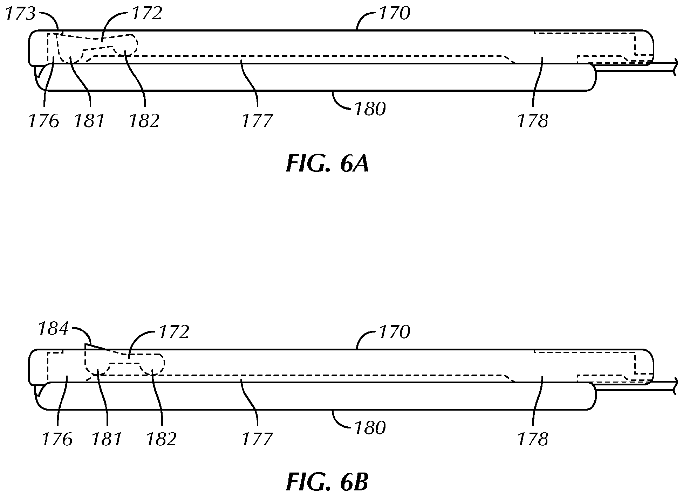

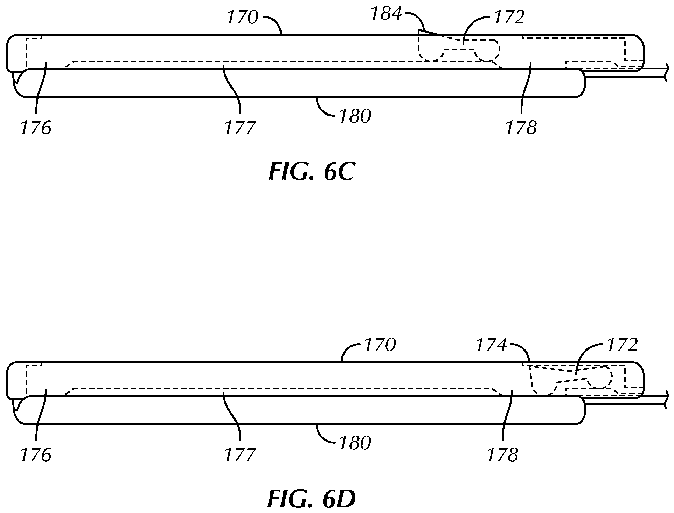

The cutter body 170 and expandable member 180 work in concert to place the cutter (e.g., cutter 172 shown in FIGS. 6A, 6B, 6C, and 6D) at the correct position within the prostatic urethra, for example facing a targeted prostatic lobe compressing the urethra. FIG. 4B depicts the expandable member 180 in an expanded configuration as compared to the unexpanded configuration depicted in FIG. 4A. The expandable member 180 is expanded to urge the cutter body 170 against the prostatic urethra when the cutter body 170 is placed at the desired position therein. The expandable member 180 ensures that there is sufficient contact between the cutter body 170 and prostate tissue to enable the cutter to cut tissue. The expansion and contraction of the expandable member 180 may be controlled via the inflation fluid conducted through the inflation lumen from the inflation port on the handle body 110 of the system 100.

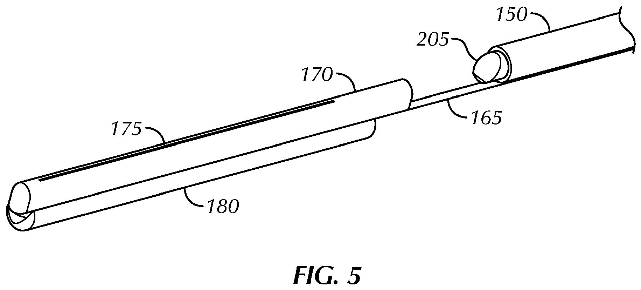

FIG. 5 is a perspective view of a distal portion of a system according to aspects of the present disclosure. In this view, the surface of the cutter body 170 that faces the tissue to be treated is shown. The cutter body 170 includes a cutter slot 175, which provides access to tissue for both the cutter (e.g., cutter 172) and an injectable substance (not pictured). The cutter slot 175 can be from about 1.0 cm to about 10 cm long, including less than about 1.0 cm or about 1.5 cm, about 2.0 cm, about 2.5 cm, about 3.0 cm, about 3.5 cm, about 4.0 cm, about 4.5 cm, about 5.0 cm, about 5.5 cm, about 6.0 cm, about 6.5 cm, about 7.0 cm, about 7.5 cm, about 8.0 cm, about 8.5 cm, about 9.0 cm, about 9.5 cm, or greater than 10.0 cm. In some examples, the length of the cutter slot 175 is preferably about 5.0 cm. The cutter slot 175 can take up a substantial portion of the tissue-facing surface of the cutter body 170, for example greater than or approximately equal to about 50%, about 55%, about 60%, about 65%, about 70%, about 75%, about 80%, about 85%, about 90%, or about 95% of the length of the cutter body 170, or more.

FIG. 5 illustrates that the articulation 165 may be flexible such that the cutter body 170 can be positioned along the same axis as the elongate member 150, for example, if required to accommodate the geometry of the relevant anatomy. Accordingly, the flexibility of the articulation 165 may configure it to assume various degrees of curvature (including zero) to accommodate a variety of anatomical features. In some examples, the articulation 165 may be biased to a particular configuration, e.g., straight or curved.

FIGS. 6A, 6B, 6C, and 6D each illustrate a side view with partial transparency of a distal portion of a system according to aspects of the present disclosure. In addition to the expandable member 180 and the cutter body 170, FIGS. 6A, 6B, 6C, and 6D illustrate a cutter 172, a cutter distal recess 176, a cutter track 177, and a cutter proximal recess 178. The cutter 172, the cutter distal recess 176, the cutter track 177, and the cutter proximal recess 178 cooperate to enable controlled cutting of tissue as the cutter 172 is pulled proximally by the cutter wire via operation of the actuator 112 on the handle body 110. FIGS. 6A, 6B, 6C, and 6D each illustrate the cutter 172 as having a distal lobe 181 and a proximal lobe 182, but other cutter shapes may be desirable and advantageous provided that they enable the cutter 172 to reside within the cutter body 170 and at least partially emerge from the cutter body 170 when the cutter 172 is actuated.

In FIG. 6A, the cutter 172 is positioned at least partially within the cutter distal recess 176 such that no part of the cutter 172 is partially emerging through the cutter slot 175. With the cutter 172 positioned at least partially within the cutter distal recess 176, the cutter body 170 can be safely introduced into tissue lumens, e.g., the urethra, and positioned at the desired therapy site, e.g., within the prostatic urethra. In some aspects, the cutter slot 175 includes a cutter slot distal lip 173 to help secure the cutter 172 within the cutter distal recess 176. That is, at least part of the cutter 172 is positioned beneath the cutter slot distal lip 173 when the cutter 172 is within the cutter distal recess 176, which helps prevent the cutter 172 from inadvertently emerging from the cutter slot 175.

In FIG. 6B, the cutter 172 has begun to travel proximally within the cutter body 170 after having been initially actuated by the actuator 112 on the handle body 110. The cutter 172 is entirely out of the cutter distal recess 176 and a portion of the cutter 172 has emerged from the cutter slot 175. In this position, the cutter 172 is configured to cut tissue as the cutter 172 moves proximally along the cutter track 177. The cutter track 177 is configured to position a cutting edge or cutting surface 184 of the cutter 172 laterally within the cutter slot 175 and also beyond the tissue-facing surface of the cutter body 170.

In FIG. 6C, the cutter 172 has nearly completed its travel proximally within the cutter body 170. In the example illustrated by FIG. 6C, the cutter track 177 positions the same amount of the cutting edge or cutting surface 184 of the cutter 172 beyond the tissue-facing surface of the cutter body 170 as the cutter 172 moves proximally along the cutter track 177. In other examples, the cutter track 177 may be configured to position more or less of the cutting edge or cutting surface 184 of the cutter 172 beyond the tissue-facing surface of the cutter body 170 as the cutter 172 moves proximally along the cutter track 177. That is, the cutter track 177 may be configured to vary the depth of cut into tissue as the cutter 172 moves proximally along the cutter track 177.

In some aspects, the depth of the cut into tissue ranges from about 2 mm to about 5 mm, including about 2.5 mm, about 3 mm, about 3.5 mm, about 4 mm, or about 4.5 mm, with a preferred depth of cut of about 2.5 mm in some examples. It may be desirable to limit the depth of cut to no greater than 5 mm in some examples to avoid cutting certain anatomical features that may be present in the region, such as the dorsal venous complex and/or the vascular plexus. In some aspects, the length of the cut into tissue ranges from about 10 mm to about 50 mm, including about 15 mm, about 20 mm, about 25 mm, about 30 mm, about 35 mm, about 40 mm, or about 45 mm, with a preferred length of cut of about 20 mm in some examples.

In FIG. 6D, the cutter 172 has moved to its farthest position proximally within the cutter body 170. At least a portion of the cutter 172 is within the cutter proximal recess 178 and none of the cutting edge or cutting surface 184 of the cutter 172 is exposed to tissue. The cutter 172 is entirely within the cutter body 170 and is positioned beneath the cutter slot proximal lip 174, which helps prevent the cutter 172 from inadvertently emerging from the cutter slot 175 while the cutter 172 is in the cutter proximal recess 178.

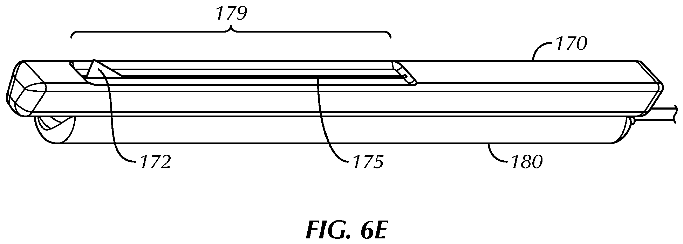

FIG. 6E is a perspective view of a distal portion of the system according to aspects of the present disclosure. FIG. 6E illustrates the presence of a cutter trough 179 in a distal section of the cutter body 170. In FIG. 6E, the cutter trough 179 is shown as having approximately the same length as the cutter slot 175, but the cutter trough 179 can have lengths greater than or less than the length of the cutter slot 175. The width of the cutter trough 179 is preferably at least two times as much as the depth of the maximum cut allowed by the configuration of the features of the cutter body 170. In any case, the cutter trough 179 should provide adequate coverage of the cut area of tissue. The cutter trough 179 may function to provide a reservoir for an injectable substance such that a sufficient amount of the injectable substance is presented to the treatment target area.

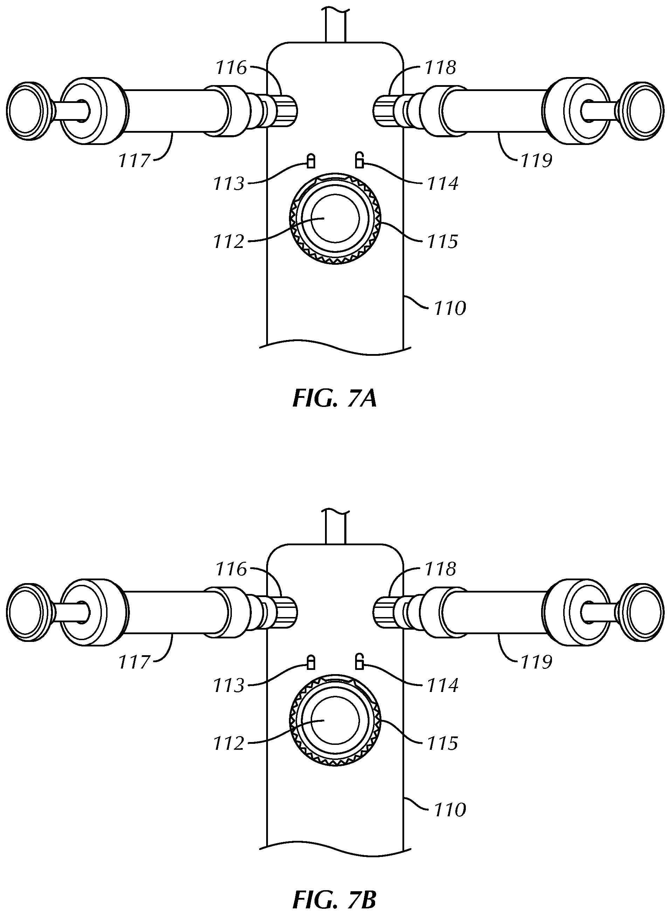

FIG. 7A and FIG. 7B are each a plan view of a proximal portion of a system according to aspects of the present disclosure. Each figure illustrates that the handle body 110 may include an actuator 112 and a lock 115. In the example illustrated in FIG. 7A and FIG. 7B, the lock 115 is a dial or knob that can be rotated from a locked position, illustrated in FIG. 7A by an indicator portion of the lock 115 being pointed to a locked indicator 113, to an unlocked position, illustrated in FIG. 7B by the indicator portion of the lock 115 being pointed to an unlocked indicator 114. The locked indicator 113 and unlocked indicator 114 can each use symbols, words, colors, and/or tactile features to indicate the state of the lock 115 to the user. The lock 115 interacts mechanically, electromechanically, electrically, or otherwise with the actuator 112 to provide a safety feature that helps prevent inadvertent actuation of the cutter.

FIG. 7A and FIG. 7B each illustrate supply devices connected with the ports on the handle body 110. An inflation supply device 117 is connected with the inflation port 116 and is configured to seal or lock with the inflation port 116 and supply inflation fluid to the system to inflate the expandable member on the distal section. Similarly, an injection supply device 119 is connected with the injection port 118 and is configured to seal or lock with the injection port 118 and supply injectable material to the system and through the cutter slot on the distal section.

In some examples of the disclosure, the handle body includes one or more pressure release valves, which may be associated with the inflation supply and/or the injection supply. A pressure release valve can mitigate or help prevent overpressurization within the system or in tissue. For example, in the event of a blockage in the inflation fluid pathway or in the injection fluid pathway, a pressure release valve can release fluid pressure after such pressure builds to a predetermined threshold thereby preventing serious damage to the system and/or patient.

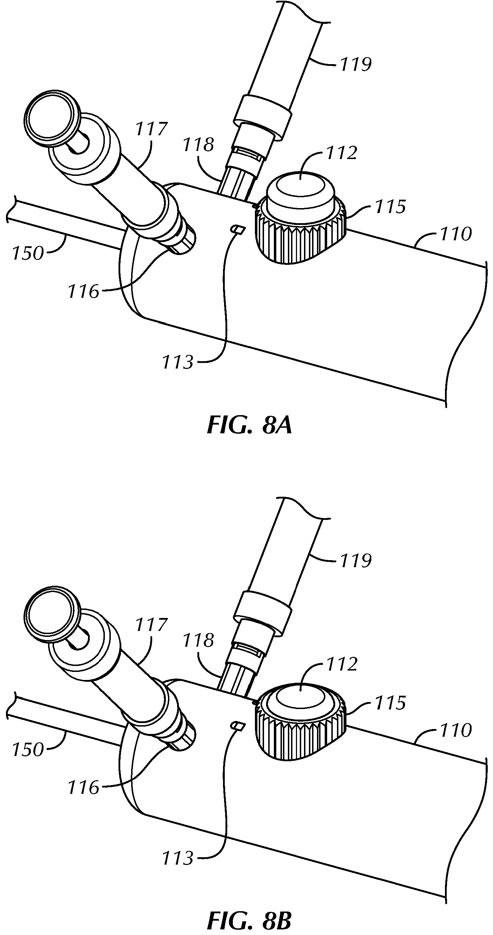

FIG. 8A and FIG. 8B are each a perspective view of a proximal portion of the system illustrating features on the proximal portion handle body 110 according to aspects of the present disclosure. In particular, FIG. 8A illustrates the actuator 112 in an initial position that corresponds with the initial, fully distal position of the cutter 172 within the cutter body 170, and FIG. 8B illustrates the actuator 112 in a final position that corresponds with the final, fully proximal position of the cutter 172 within the cutter body 170. Thus, FIG. 8A and FIG. 8B illustrate that the actuator 112 may be pushed toward the handle body 110 to actuate the cutter motion. The cutter motion can be driven by mechanical, electrical, electromechanical, hydraulic, pneumatic, or other forces. In some aspects, the cutter 172 is configured to be resettable to its initial position or an intermediate position. After being reset, the cutter 172 can be actuated again to make another cut. This resetting process can occur multiple times such that additional cuts can be made in tissue, optionally after the device has been repositioned to a new treatment target area or rotated within the existing treatment target area. Repositioning the device may require contraction of the expandable member 180.

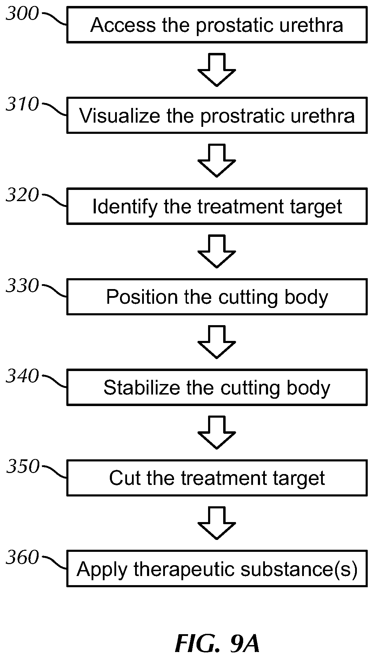

FIG. 9A is a flowchart illustrating steps in a method for using a system disclosed herein (e.g., system 100) according to aspects of the present disclosure. The example method shows steps that may be utilized, in the particular order depicted or in one or more different sequences, by embodiments of the systems and devices described herein. Fewer or more steps may be implemented in accordance with variations of the method shown in FIG. 9A and consistent with the remainder of the present disclosure.

As an initial step 300 in the method, a user gains access to the prostatic urethra by inserting the system directly into the patient's urethra or via an intermediary device, such as an access sheath, an introducer, a guidewire, or a combination thereof, that has been placed partially within the patient's urethra. In a second step 310 in the method, an endoscope connected with the system is used to visualize the prostatic urethra and the tissue regions therein. The endoscope can be inserted into and coupled with the system prior to the system being inserted within the patient or after the system has been inserted within the patient. The endoscope is connected with a viewing system.

In a third step 320 in the method, the endoscope connected with the system is used to identify the treatment target. In some aspects of the disclosure, the treatment target is a commissure in the prostatic urethra, and in some cases the target is the anterior commissure in the prostatic urethra. In a fourth step 330 in the method, the cutting body is positioned against the treatment target. Positioning the cutting body may involve rotation of the system to align the cutting body radially with the treatment target, axial movement of the system to align the cutting body longitudinally with the treatment target, and/or lateral movement of the system. These movements can take place in any combination or sequence. In some aspects, a user may visualize the prostatic urethra, identify the treatment target, and/or position the cutting body by positioning the endoscope just past sphincter such that only the proximal portion of the cutter body is visible.

Referring still to FIG. 9A and in a fifth step 340 of the method, the cutting body is stabilized against the treatment target. When the cutting body is in the desired position against the treatment target, the expandable member can be expanded to stabilize the cutting body in its position. Alternatively, the cutting body can be positioned proximate the treatment target, but not against the treatment target, such that expansion of the expandable member moves the cutting body from its position proximate the treatment target to a position against the treatment target. The expandable member can be expanded by the user employing the inflation supply device connected with the inflation port on the handle body. In one example, the user can depress the plunger of an inflation fluid syringe while observing the distal section of the system through the endoscope to ensure proper positioning and stabilization of the cutter body against the treatment target. In other examples, other imaging methods, such as fluoroscopy, can be used with a contrast-bearing fluid to visualize the expansion of the expandable member and the positioning and stabilizing of the cutting body.

In a sixth step 350 of the method, the cutter is deployed to cut tissue in the treatment target. In one example, the cutter is actuated by the user unlocking the lock on the handle body and pressing the actuator. The cutter moves from its distal position entirely within the cutter body to a position where a cutting edge or surface protrudes from the cutter body and then moves proximally to cut the tissue of the treatment target and then reaches a proximal position where the cutting edge or surface is again entirely within the cutter body. In some examples, the cutter can be actuated multiple times such that the cutter now moves distally to return to its original position and thereby cut any tissue in the treatment target that it encounters. Still further, the cutter may be actuated yet again to make a third pass through the treatment target and so on. In some aspects, the cutter is configured to be resettable to its initial position or an intermediate position. After being reset, the cutter can be actuated again to make another cut. This resetting process can occur multiple times such that additional cuts can be made in tissue, optionally after the device has been repositioned to a new treatment target area or rotated within the existing treatment target area. Repositioning the device may require contraction of the expandable member. In these cases where the cutter is reset, the method can cycle back to any one of steps 310 (visualizing the prostatic urethra), 320 (identifying the treatment target), or 330 (positioning the cutter body).

Referring still to FIG. 9A and in a seventh step 360 of the method, one or more therapeutic substances may be applied to the treatment target. Such one or more therapeutic substances may be applied through the cutter slot via the injection supply device connected with the injection port on the handle body of the device. The injection supply device can contain one or more therapeutic substances and, in some examples, another injection supply device containing one or more other therapeutic substances can subsequently be connected to the injection port for application of such one or more other therapeutic substances to the treatment target.

The therapeutic substance(s) that can be used in the system can be any therapeutic agent or substance. In some preferred aspects, the therapeutic substance is a fibrin gel, which can act as a hemostatic agent and a tissue scaffold to maintain the physical position and arrangement of the tissue as it heals. The fibrin gel may be used on its own or in conjunction with other therapeutic substances mixed in with the fibrin gel or delivered prior to or subsequent to the application of the fibrin gel.

Examples of other substances that are especially useful are lipophilic substantially water insoluble drugs, such as paclitaxel, rapamycin, daunorubicin, doxorubicin, lapachone, vitamin D2 and D3 and analogues and derivatives thereof. Other drugs that may be useful include, without limitation, glucocorticoids (e.g., dexamethasone, betamethasone), hirudin, angiopeptin, aspirin, growth factors, antisense agents, anti-cancer agents, anti-proliferative agents, oligonucleotides, and, more generally, anti-platelet agents, anti-coagulant agents, anti-mitotic agents, antioxidants, anti-metabolite agents, anti-chemotactic, and anti-inflammatory agents. Some drugs that are considered particularly suitable for combatting inflammation are corticosteroids such as, budesonide, flunisolide, triamcinolone, beclomethasone, fluticasone, mometasone, mometasone furoate, dexamethasone, hydrocortisone, methylprednisolone, prednisone, cotisone, betamethasone, triamcinolone acetonide, or the like. Also useful are polynucleotides, antisense, RNAi, or siRNA, for example, that inhibit inflammation and/or smooth muscle cell or fibroblast proliferation. Anti-platelet agents can include drugs such as aspirin and dipyridamole. Aspirin is classified as an analgesic, antipyretic, anti-inflammatory and anti-platelet drug. Dipyridamole is a drug similar to aspirin in that it has anti-platelet characteristics. Dipyridamole is also classified as a coronary vasodilator. Anti-coagulant agents can include drugs such as heparin, protamine, hirudin and tick anticoagulant protein. Anti-oxidant agents can include probucol. Anti-proliferative agents can include drugs such as amlodipine and doxazosin. Anti-mitotic agents and anti-metabolite agents that can be used include drugs such as methotrexate, azathioprine, vincristine, vinblastine, 5-fluorouracil, adriamycin, and mutamycin. Antibiotic agents for use in the system include penicillin, cefoxitin, oxacillin, tobramycin, and gentamicin. Suitable antioxidants for use in the system include probucol. Additionally, genes or nucleic acids, or portions thereof can be used as the therapeutic agent. Furthermore, collagen-synthesis inhibitors, such as tranilast, can be used as a therapeutic agent. Other drugs for use in the system also include everolimus, somatostatin, tacrolimus, roxithromycin, dunaimycin, ascomycin, bafilomycin, erythromycin, midecamycin, josamycin, concanamycin, clarithromycin, troleandomycin, folimycin, cerivastatin, simvastatin, lovastatin, fluvastatin, rosuvastatin, atorvastatin, pravastatin, pitavastatin, vinblastine, vincristine, vindesine, vinorelbine, etoposide, teniposide, nimustine, carmustine, lomustine, cyclophosphamide, 4-hydroxycyclophosphamide, estramustine, melphalan, ifosfamide, trofosfamide, chlorambucil, bendamustine, dacarbazine, busulfan, procarbazine, treosulfan, temozolomide, thiotepa, daunorubicin, doxorubicin, aclarubicin, epirubicin, mitoxantrone, idarubicin, bleomycin, mitomycin, dactinomycin, methotrexate, fludarabine, fludarabine-5'-dihydrogenphosphate, cladribine, mercaptopurine, thioguanine, cytarabine, fluorouracil, gemcitabine, capecitabine, docetaxel, carboplatin, cisplatin, oxaliplatin, amsacrine, irinotecan, topotecan, hydroxycarbamide, miltefosine, pentostatin, aldesleukin, tretinoin, asparaginase, pegaspargase, anastrozole, exemestane, letrozole, formestane, aminoglutethimide, adriamycin, azithromycin, spiramycin, cepharantin, smc proliferation inhibitor-2w, epothilone A and B, mitoxantrone, azathioprine, mycophenolatmofetil, c-myc-antisense, b-myc-antisense, betulinic acid, camptothecin, lapachol, beta.-lapachone, podophyllotoxin, betulin, podophyllic acid 2-ethylhydrazide, molgramostim (rhuGM-CSF), peginterferon a-2b, lenograstim (r-HuG-CSF), filgrastim, macrogol, dacarbazine, basiliximab, daclizumab, selectin (cytokine antagonist), CETP inhibitor, cadherines, cytokinin inhibitors, COX-2 inhibitor, NFkB, angiopeptin, ciprofloxacin, camptothecin, fluoroblastin, monoclonal antibodies, which inhibit the muscle cell proliferation, bFGF antagonists, probucol, prostaglandins, 1,11-dimethoxycanthin-6-one, 1-hydroxy-11-methoxycanthin-6-one, scopoletin, colchicine, NO donors such as pentaerythritol tetranitrate and syndnoeimines, S-nitrosoderivatives, tamoxifen, staurosporine, beta.-estradiol, a-estradiol, estriol, estrone, ethinylestradiol, fosfestrol, medroxyprogesterone, estradiol cypionates, estradiol benzoates, tranilast, kamebakaurin and other terpenoids, which are applied in the therapy of cancer, verapamil, tyrosine kinase inhibitors (tyrphostines), cyclosporine A, 6-a-hydroxy-paclitaxel, baccatin, taxotere and other macrocyclic oligomers of carbon suboxide (MCS) and derivatives thereof, mofebutazone, acemetacin, diclofenac, lonazolac, dapsone, o-carbamoylphenoxyacetic acid, lidocaine, ketoprofen, mefenamic acid, piroxicam, meloxicam, chloroquine phosphate, penicillamine, hydroxychloroquine, auranofin, sodium aurothiomalate, oxaceprol, celecoxib, .beta.-sitosterin, ademetionine, myrtecaine, polidocanol, non ivamide, levomenthol, benzocaine, aescin, ellipticine, D-24851 (Calbiochem), colcemid, cytochalasin A-E, indanocine, nocodazole, S 100 protein, bacitracin, vitronectin receptor antagonists, azelastine, guanidyl cyclase stimulator tissue inhibitor of metal proteinase-1 and -2, free nucleic acids, nucleic acids incorporated into virus transmitters, DNA and RNA fragments, plasminogen activator inhibitor-1, plasminogen activator inhibitor-2, antisense oligonucleotides, VEGF inhibitors, IGF-1, active agents from the group of antibiotics such as cefadroxil, cefazolin, cefaclor, cefotaxim, tobramycin, gentamycin, penicillins such as dicloxacillin, oxacillin, sulfonamides, metronidazol, antithrombotics such as argatroban, aspirin, abciximab, synthetic antithrombin, bivalirudin, coumadin, enoxaparin, desulphated and N-reacetylated heparin, tissue plasminogen activator, GpIIb/IIIa platelet membrane receptor, factor Xa inhibitor antibody, heparin, hirudin, r-hirudin, PPACK, protamin, prourokinase, streptokinase, warfarin, urokinase, vasodilators such as dipyramidole, trapidil, nitroprussides, PDGF antagonists such as triazolopyrimidine and seramin, ACE inhibitors such as captopril, cilazapril, lisinopril, enalapril, losartan, thiol protease inhibitors, prostacyclin, vapiprost, interferon a, .beta and y, histamine antagonists, serotonin blockers, apoptosis inhibitors, apoptosis regulators such as p65 NF-kB or Bcl-xL antisense oligonucleotides, halofuginone, nifedipine, tranilast, molsidomine, tea polyphenols, epicatechin gallate, epigallocatechin gallate, Boswellic acids and derivatives thereof, leflunomide, anakinra, etanercept, sulfasalazine, etoposide, dicloxacillin, tetracycline, triamcinolone, mutamycin, procainamide, retinoic acid, quinidine, disopyramide, flecamide, propafenone, sotalol, amidorone, natural and synthetically obtained steroids such as bryophyllin A, inotodiol, maquiroside A, ghalakinoside, mansonine, strebloside, hydrocortisone, betamethasone, dexamethasone, non-steroidal substances (NSAIDS) such as fenoprofen, ibuprofen, indomethacin, naproxen, phenylbutazone and other antiviral agents such as acyclovir, ganciclovir and zidovudine, antimycotics such as clotrimazole, flucytosine, griseofulvin, ketoconazole, miconazole, nystatin, terbinafine, antiprozoal agents such as chloroquine, mefloquine, quinine, moreover natural terpenoids such as hippocaesculin, barringtogenol-C21-angelate, 14-dehydroagrostistachin, agroskerin, agrostistachin, 17-hydroxyagrostistachin, ovatodiolids, 4,7-oxycycloanisomelic acid, baccharinoids B1, B2, B3 and B7, tubeimoside, bruceanol A, B and C, bruceantinoside C, yadanziosides N and P, isodeoxyelephantopin, tomenphantopin A and B, coronarin A, B, C and D, ursolic acid, hyptatic acid A, zeorin, iso-iridogermanal, maytenfoliol, effusantin A, excisanin A and B, longikaurin B, sculponeatin C, kamebaunin, leukamenin A and B, 13,18-dehydro-6-a-senecioyloxychaparrin, taxamairin A and B, regenilol, triptolide, moreover cymarin, apocymarin, aristolochic acid, anopterin, hydroxyanopterin, anemonin, protoanemonin, berberine, cheliburin chloride, cictoxin, sinococuline, bombrestatin A and B, cudraisoflavonei A, curcumin, dihydronitidine, nitidine chloride, 12-beta-hydroxypregnadien-3,20-dione, bilobol, ginkgol, ginkgolic acid, helenalin, indicine, indicine-N-oxide, lasiocarpine, inotodiol, glycoside 1a, podophyllotoxin, justicidin A and B, larreatin, malloterin, mallotochromanol, isobutyrylmallotochromanol, maquiroside A, marchantin A, maytansine, lycoridicin, margetine, pancratistatin, liriodenine, bisparthenolidine, oxoushinsunine, aristolactam-All, bisparthenolidine, periplocoside A, ghalakinoside, ursolic acid, deoxypsorospermin, psychorubin, ricin A, sanguinarine, manwu wheat acid, methylsorbifolin, sphatheliachromen, stizophyllin, mansonine, strebloside, akagerine, dihydrousambarensine, hydroxyusambarine, strychnopentamine, strychnophylline, usambarine, usambarensine, berberine, liriodenine, oxoushinsunine, daphnoretin, lariciresinol, methoxylariciresinol, syringaresinol, umbelliferon, afromoson, acetylvismione B, desacetylvismione A, and vismione A and B.

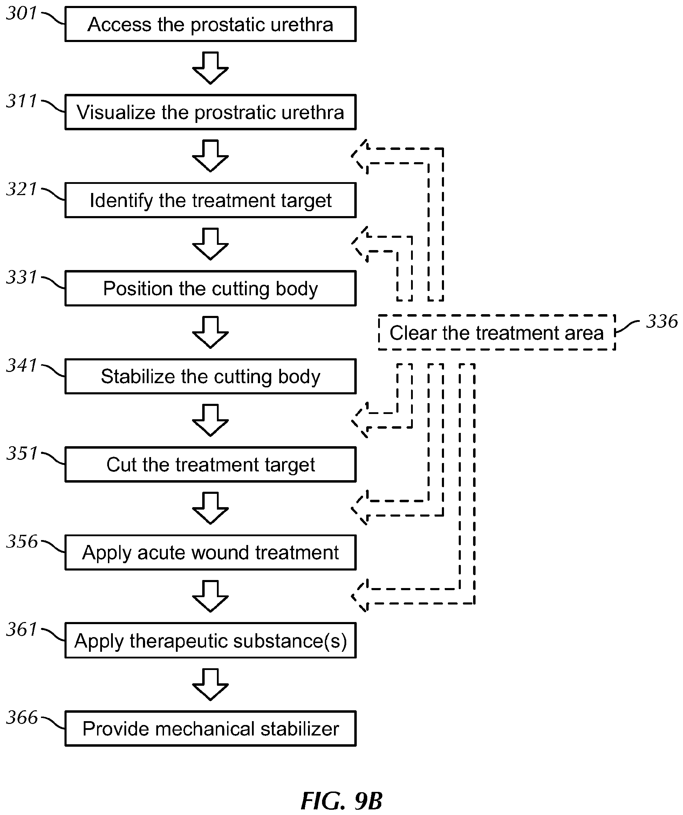

FIG. 9B is a flowchart illustrating steps in another method for using a system disclosed herein (e.g., system 100) according to aspects of the present disclosure. The example method shows steps that may be utilized, in the particular order depicted or in one or more different sequences, by embodiments of the systems and devices described herein. Fewer or more steps may be implemented in accordance with variations of the method shown in FIG. 9B and consistent with the remainder of the present disclosure.

As an initial step 301 in the method, a user gains access to the prostatic urethra via inserting the system directly into the patient's urethra or via an intermediary device, such as an access sheath, an introducer, a guidewire, or a combination thereof, that has been placed partially within the patient's urethra. In a second step 311 in the method, the endoscope connected with the system is used to visualize the prostatic urethra and the tissue regions therein. In a third step 321 in the method, the endoscope connected with the system is used to identify the treatment target. In a fourth step 331 in the method, the cutting body is positioned against the treatment target. In a fifth step 341 of the method, the cutting body is stabilized against the treatment target. In a sixth step 351 of the method, the cutter is deployed to cut tissue in the treatment target. In a seventh step 356 of the method, an acute wound treatment is applied to the treatment target, including, but not limited to, hot water treatment, light treatment, air treatment, heat treatment, energy treatment (such as with radio-frequency energy) to control bleeding and/or otherwise prepare the cut tissue for the next steps. The acute wound treatment step is intended to address the acute tissue response to the cutting step. In an eighth step 361 of the method, one or more therapeutic substances is applied to the treatment target. In a ninth step 366 of the method, a mechanical stabilizer is applied to the treatment target to maintain the position and/or arrangement of the cut tissue. In cases where the cut tissue may be disposed to knit together after being cut or otherwise reverse or diminish the therapeutic gains made via cutting the tissue, one or more mechanical stabilizers, including but not limited to tissue anchors, tissue retractors, implants, stents, catheters, and the like, may be placed in or near the treatment target area. These mechanical stabilizers may be temporary or permanent. In some aspects, it may be desirable to place a catheter in the patient as is commonly done in similar procedures.

Referring still to FIG. 9B, in an optional step 336 of the method the treatment target area may be cleared via irrigation and/or suction. This optional step 336 can be used one or more times in the method and at various points in the method, such as between steps 311 and 321, between steps 321 and 331, between steps 331 and 341, between steps 341 and 351, between steps 351 and 356, and between steps 356 and 361. In some aspects, the optional step 336 can be used between steps 351 and 361 if step 356 is not used in the method.

Among the desirable features of the system and method disclosed herein is the ability to control the location, depth, and length of the cut into tissue. Generally speaking, while the system and method provide the ability to cut various and multiple locations related to the treatment of BPH, some locations within the anatomy may yield a greater therapeutic effect than others. These locations of greatest effect may vary from patient to patient or may be generally understood. Embodiments of the systems and methods of the disclosure expressly include identifying the desirability of a treatment target location based on a given patient's anatomy, disease state, and other relevant factors.

Other applications of the system are included in the method of use. For example, it may be desirable to use the system to cut "high tone" bladder necks to make it easier for a patient to urinate. In such a use, the methods of FIG. 9A and FIG. 9B are applicable.

FIG. 10 is a perspective view of another system according to aspects of the present disclosure. The system 400 is illustrated as coupled to endoscope body 500 at the proximal end of the handle body 410 of the system 400. The handle body 410 in the example shown includes an actuator 412 operatively connected to the distal section 460 of the system 400. Specifically, the actuator 412 can be operatively connected to a deployment or delivery mechanism in the distal section 460 of the system 400, and may thus be referred to as the "deployment actuator" in various embodiments. The handle body 400 may also include a lock 415 engaged with the actuator 412 to lock or unlock the actuator 412. The lock 415 engages the actuator 412 to lock it in the sense that the lock 415 prevents the actuator 412 from being operated when in a locked position. Conversely, the lock 415 can disengage the actuator 412 to unlock it and allow the actuator to be operated from an unlocked position.

In the example illustrated in FIG. 10, the lock 415 is a dial or knob that can be rotated from a locked position in the same or similar manner as the lock 115 depicted in FIGS. 7A and 7B. A locked indicator 413 and an unlocked indicator 414 can each use symbols, words, colors, and/or tactile features to indicate the state of the lock 415 to the user, in the same or similar manner as the locked indicator 113 and unlocked indicator 114 also depicted in FIGS. 7A and 7B. The lock 415 interacts mechanically, electromechanically, electrically, or otherwise with the actuator 412 to provide a safety feature that helps prevent inadvertent actuation of the delivery mechanism.

As shown in FIG. 10, the handle body 410 may include an inflation port 416 configured to transport fluid through an inflation lumen within the handle body 410 and within the elongate member 450 of the system to the distal section 460 of the system 400. The system 400 includes an elongate member 450 that connects the handle body 410 with the distal section 460. The elongate member 450 includes an endoscope lumen for the elongated portion of the endoscope that is intended for use within a patient's body. The elongate member 450 also includes the inflation lumen previously described. And in some examples of the system the elongate member includes an actuation wire within the elongate member 450 but separate from the inflation lumen. The elongate member 450 may be rigid, semi-rigid, or semi-flexible. The elongate member 450 is configured to be insertable within the urethra of a patient and is composed of materials suitable for medical use.

FIG. 11 is a perspective view of a system according to aspects of the present disclosure. An inflation supply device 417 is connected with the inflation port 416 and is configured to seal or lock with the inflation port 416 and supply inflation fluid to the system to inflate an expandable member 480 on the distal section. In some examples, the handle body may include one or more pressure release valves, which may be associated with the inflation supply. A pressure release valve can mitigate or help prevent overpressurization within the system or in tissue. For example, in the event of a blockage in the inflation fluid pathway, a pressure release valve can release fluid pressure after such pressure builds to a predetermined threshold thereby preventing damage to the system and/or patient.

FIG. 10 and FIG. 11 each illustrate that the elongate member 450 of the system is connected via an articulation 465 (or "articulated section") to the distal section 460. The endoscope distal end 505 can be seen at the distal end of the elongate member 450. In this non-limiting example, the endoscope distal end 505 is a 30-degree cystoscope. Additional endoscopes may be used.

The articulation 465, which is more flexible than the elongate member 450, may be configured to curve at a pre-formed angle with respect to the long axis of the elongate member 450. The pre-formed curve in the articulation 465 may be configured to generally match the typical anatomical curvature of the urethra near the prostate and bladder neck. The articulation 465 may be flexible to enable the articulation 465 to conform to the curvature of the urethra and position the distal section 460 in the prostatic urethra without puncturing the urethral wall. The articulation 465 presents the distal section 460 within easy view of the 30-degree cystoscope. The articulation 465 can be formed from plastics, shape memory materials, or other flexible to semi-flexible materials suitable for medical use. The flexibility of the articulation 465 allows for comparatively easier navigation of the urethra as compared to an entirely rigid device in addition to presenting the distal section 460 at a desirable angle within the anatomy with respect to the long axis of the elongate member 450. The articulation 465 may include the inflation lumen and the actuation wire.

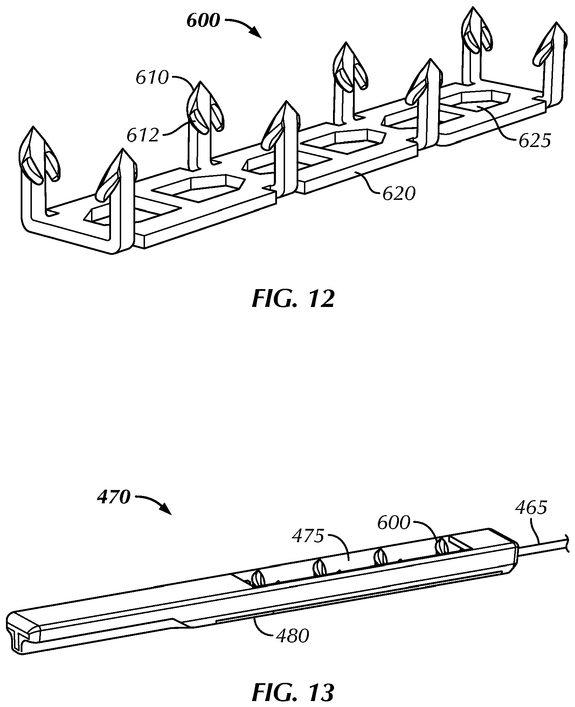

FIG. 12 is a perspective view of a tissue manipulation structure 600 according to aspects of the present disclosure. The tissue manipulation structure 600 includes a tissue stabilizing member 620, which can be configured to both manipulate and stabilize tissue. The tissue stabilizing member 620 can stabilize the tissue in the commissure such that the commissure is held open more so than without the tissue stabilizing member 620, for instance such that the commissure is help open longer or to a greater extent than without the tissue stabilizing member 620. This stabilized opening in tissue at the commissure provides the beginning of a channel for urine to begin to flow and then hydraulic pressure opens the remainder of the prostatic urethra. In some aspects, more than one tissue stabilizing member 620 can be delivered to the prostatic urethra to manipulate and stabilize tissue and create some or all of a flow channel.

Still referring to FIG. 12, the tissue stabilizing member 620 can have a length in the range of about 10 mm to about 50 mm, and in some examples has a length in the range of about 25 mm to about 30 mm. In other examples, a plurality of tissue stabilizing members 620 of comparatively shorter length can be used with a single elongate member. In accordance with such examples, one or more of the tissue stabilizing members 620 can have a length in the range of from about 5 mm to about 15mm, including about 6 mm, about 7 mm, about 8 mm, about 9 mm, about 10 mm, about 11 mm, about 12 mm, about 13 mm, or about 14 mm. The tissue stabilizing member 620 can have a width in the range of about 3 mm to about 10 mm, including about 4 mm, about 5 mm, about 6 mm, about 7 mm, about 8 mm, or about 9 mm, with a preferred width in some examples in the range of about 5 mm to about 6 mm.

The tissue stabilizing member 620 can be solid, perforated, fenestrated, or can have some solid portions and some perforated and/or fenestrated portions. FIG. 12 illustrates the tissue stabilizing member 620 as having six fenestrations 625. In some aspects, the fenestrations 625 may facilitate tissue ingrowth and/or tissue healing. The tissue stabilizing member 620 can have a height in the range of about 1 mm to about 3 mm and preferably has a height of about 1 mm.

FIG. 12 illustrates that the tissue manipulation structure 600 can include one or more tissue fixation members 610. FIG. 12 depicts eight tissue fixation members 610 present in the tissue manipulation structure 600. The tissue fixation members 610 may be arranged in a pattern such that a set of tissue fixation members 610 is present on each side of the commissure, each set runs generally along the longitudinal axis of the prostatic urethra, and each set penetrates tissue of a prostatic lobe. The term "side of the commissure" may refer to a portion of one of the lateral lobes that abut to form the commissure. Thus, one set of tissue fixation members 610 may penetrate one of the lateral lobes and the other set of tissue fixation members 610 may penetrate the other lateral lobe. The tissue fixation members 610 in each set may be arranged equidistant from each other across the tissue stabilizing member 620, or the distance across the tissue stabilizing member 620 between tissue fixation members 610 may vary. For example, the pair of sets of tissue fixation members 610 across the tissue stabilizing member 620 can form a "V" shape or a "zig-zag" shape or other shapes useful to straddle the commissure. In some aspects, there is not a tissue fixation member 610 of one set directly across the tissue stabilizing member 620 from a member of the other set. In aspects in which multiple tissue manipulation structures 600 are deployed to the prostatic urethra, the arrangement of tissue fixation members 610 on a tissue stabilizing member 620 can vary among the multiple tissue manipulation structures 600.

The tissue fixation members 610 can have a height in the range of from about 2 mm to about 6 mm, including about 2.5 mm, about 3 mm, about 3.5 mm, about 4 mm, about 4.5 mm, about 5 mm, or about 5.5 mm, with a height of about 3 mm being preferred in some examples. Thus, the overall height of the tissue manipulation structure 600 can vary from about 3 mm to about 8 mm, inclusive, and may be preferably about 4 mm in some examples. The tissue fixation members 610 may be of uniform height or nonuniform height. The heights of the tissue fixation members 610 may vary in any suitable pattern among the tissue fixation members 610 of a set and between the sets of tissue fixation members 610.

The tissue fixation members 610 include one or more features to enable stable fixation in tissue. FIG. 12 illustrates the tissue fixation members 610 as each having two tissue fixation barbs 612, although more or fewer tissue fixation barbs 612 may be present on each tissue fixation members 610. The number of tissue fixation barbs 612, or any fixation feature, present on an individual tissue fixation member 610 may be the same or different from the number of tissue fixation barbs 612, or any fixation feature, present on any other tissue fixation member 610 of the same tissue manipulation structure 600. The tissue fixation barbs 612 may have varying flexibility as compared to the prostatic tissue such that the tissue fixation barbs 612 may flex towards the tissue fixation members 610 when being inserted into tissue or the tissue fixation barbs 612 may remain stationary when being inserted into tissue. An important aspect of the tissue fixation barbs 612 is that they may resist movement of the tissue fixation member 610 out from tissue once tissue has been penetrated by the tissue fixation member 610.

It may be desirable in some examples to limit the depth of penetration into tissue to no greater than about 5 mm to avoid damaging certain anatomical features that may be present in the region, such as the dorsal venous complex and/or the vascular plexus.

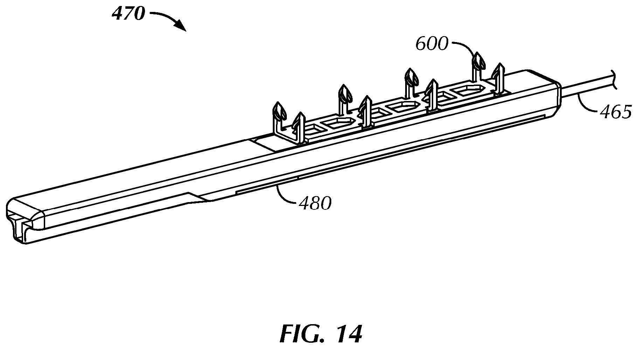

FIG. 13 and FIG. 14 are perspective views of a distal section of an example system according to aspects of the present disclosure. The surface of the delivery body 470 that faces the tissue to be treated is shown. The delivery body 470 includes a delivery body chamber 475, from which the tissue manipulation structure 600 can be delivered to tissue. The delivery body chamber 475 is configured to be a size suitable for the delivery of one or more tissue manipulation structures 600. Thus, the delivery body chamber 475 can have a length in the range of about 10 mm to about 50 mm, and preferably has a length in the range of about 25 mm to about 30 mm. The delivery body chamber 475 can have a width in the range of about 3 mm to about 10 mm, and preferably has a width in the range of about 5 mm to about 6 mm. And the delivery body chamber 475 can have a depth varying from about 4 mm to about 10 mm and may be preferably about 5 mm in some examples. The delivery body chamber 475 can take up a substantial portion of the tissue-facing surface of the delivery body 470, for example about 50% of the length of the tissue-facing surface or more, such as about 55%, about 60%, about 65%, about 70%, about 75%, about 80%, about 85%, about 90%, or about 95%, or more.

The delivery body 470 and expandable member 480 may work in concert to place the tissue manipulation structure 600 at the correct position within the prostatic urethra. The expandable member 480 is expanded to urge the delivery body 470 against the prostatic urethra when the delivery body 470 has been placed at the desired position therein. The expandable member 480 ensures that there is sufficient contact between the delivery body 470 and prostate tissue to enable accurate placement of the tissue manipulation structure 600 about the commissure. The expansion and contraction of the expandable member 480 may be controlled via the inflation fluid conducted through the inflation lumen from the inflation port on the handle body of the system.

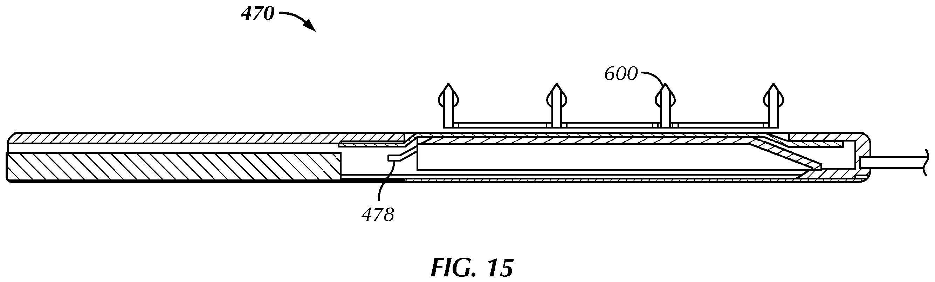

FIG. 15 is a side view with partial transparency of a distal portion of a system according to aspects of the present disclosure. As shown, a delivery body mechanism 478 actuated via an actuation wire, for example, operates to deliver the tissue manipulation structure 600. In one example, the delivery body mechanism 478 is a ramped feature that, when pulled proximally within the delivery body 470 by the actuation wire, forces the tissue manipulation structure 600 out of the delivery body chamber 475. It may be desirable to force the tissue manipulation structure 600 out of the delivery body 470 rapidly enough to cleanly penetrate prostatic tissue, for instance such that undesired lateral tearing of the tissue is avoided. The delivery motion can be driven by a variety of mechanisms or forces, including mechanical, electrical, electromechanical, hydraulic, and/or pneumatic forces. In some aspects, the delivery body mechanism 478 may be configured to be resettable to its initial position or an intermediate position. After being reset, the delivery body mechanism 478 can be actuated again to deliver another tissue manipulation structure 600. This resetting process can occur multiple times such that additional tissue manipulation structures 600 can be placed in tissue, optionally after the device has been repositioned to a new treatment target area or within the existing treatment target area. Repositioning the device may require contraction of the expandable member 480.

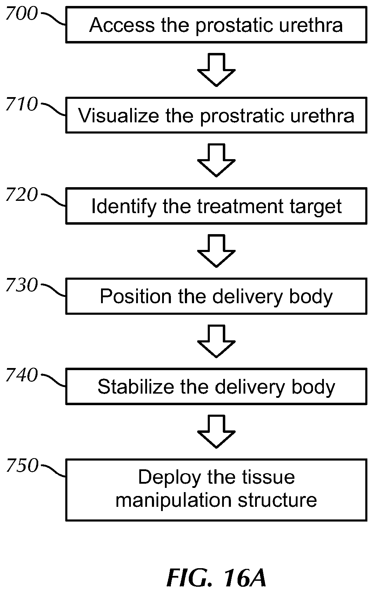

FIG. 16A is a flowchart illustrating steps in a method for using a system disclosed herein (e.g., system 400) according to aspects of the present disclosure. The example method shows steps that may be utilized, in the particular order depicted or in one or more different sequences, by embodiments of the systems and devices described herein. Fewer or more steps may be implemented in accordance with variations of the method shown in FIG. 16A and consistent with the remainder of the present disclosure.

As an initial step 700 in the method, a user gains access to the prostatic urethra via inserting the system directly into the patient's urethra or via an intermediary device, such as an access sheath, an introducer, a guidewire, or a combination thereof, that has been placed partially within the patient's urethra. In a second step 710 in the method, the endoscope connected with the system is used to visualize the prostatic urethra and the tissue regions therein. The endoscope can be inserted into and coupled with the system prior to the system being inserted within the patient or after the system has been inserted within the patient. The endoscope is connected with a viewing system. In a third step 720 in the method, the endoscope connected with the system is used to identify the treatment target. In some aspects of the disclosure, the treatment target is a commissure in the prostatic urethra and in some cases the target is the anterior commissure in the prostatic urethra. In a fourth step 730 in the method, the delivery body is positioned against the treatment target. Positioning the delivery body may involve rotation of the system to align the delivery body radially with the treatment target, axial movement of the system to align the delivery body longitudinally with the treatment target, and/or lateral movement of the system. These movements can take place in any combination or sequence. In some aspects, a user may visualize the prostatic urethra, identify the treatment target, and/or position the delivery body by positioning the endoscope just past the sphincter such that only the proximal portion of the delivery body is visible.

Referring still to FIG. 16A and in a fifth step 740 of the method, the delivery body is stabilized against the treatment target. When the delivery body is in the desired position against the treatment target, the expandable member can be expanded to stabilize the delivery body in its position. Alternatively, the delivery body can be positioned proximate the treatment target, but not against the treatment target, such that expansion of the expandable member moves the delivery body from its position proximate the treatment target to a position against the treatment target. The expandable member can be expanded by the user employing the inflation supply device connected with the inflation port on the handle body. In one example, the user can depress the plunger of an inflation fluid syringe while observing the distal section of the system through the endoscope to ensure proper positioning and stabilization of the delivery body against the treatment target. In another example, other imaging methods, such as fluoroscopy, can be used with a contrast-bearing fluid to visualize the expansion of the expandable member and the positioning and stabilizing of the delivery body.

In a sixth step 750 of the method, the tissue manipulation structure is deployed to manipulate and stabilize tissue in the treatment target. In one example, the tissue manipulation structure is deployed by the user unlocking the lock on the handle body and pressing the actuator. In some aspects, the actuator is configured to be resettable to its initial position or an intermediate position. After being reset, the actuator can be actuated again to deploy another tissue manipulation structure. This resetting process can occur multiple times such that additional tissue manipulation structures can be placed in tissue, optionally after the device has been repositioned to a new treatment target area or rotated within the existing treatment target area. Repositioning the device may require contraction of the expandable member. In these cases where the actuator is reset, the method can cycle back to any one of steps 710 (visualizing the prostatic urethra), 720 (identifying the treatment target), or 730 (positioning the delivery body).

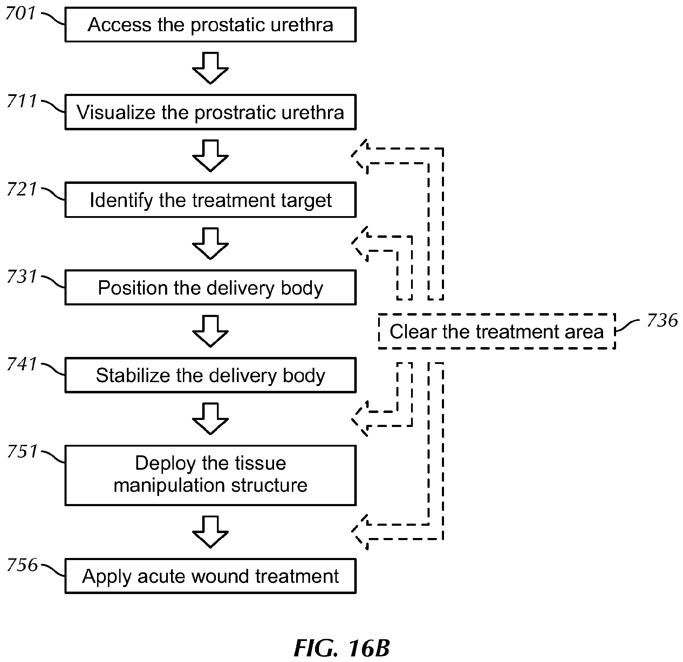

FIG. 16B is a flowchart illustrating steps in another method for using a system disclosed herein (e.g., system 400) according to aspects of the present disclosure. The example method shows steps that may be utilized, in the particular order depicted or in one or more different sequences, by embodiments of the systems and devices described herein. Fewer or more steps may be implemented in accordance with variations of the method shown in FIG. 16B and consistent with the remainder of the present disclosure.

As an initial step 701 in the method, a user gains access to the prostatic urethra via inserting the system directly into the patient's urethra or via an intermediary device, such as an access sheath, an introducer, a guidewire, or a combination thereof, that has be placed partially within the patient's urethra. In a second step 711 in the method, the endoscope connected with the system is used to visualize the prostatic urethra and the tissue regions therein. In a third step 721 in the method, the endoscope connected with the system is used to identify the treatment target. In a fourth step 731 in the method, the delivery body is positioned against the treatment target. In a fifth step 741 of the method, the delivery body is stabilized against the treatment target. In a sixth step 751 of the method, the tissue manipulation structure is deployed to manipulate and stabilize tissue in the treatment target. In a seventh step 756 of the method, an acute wound treatment may be applied to the treatment target, including, but not limited to, hot water treatment, light treatment, air treatment, heat treatment, energy treatment (such as with radio-frequency energy) to control bleeding and/or otherwise treat the manipulated tissue. The acute wound treatment step is intended to address the acute tissue response to the deployment step. In some aspects, it may be desirable to place a catheter in the patient as is commonly done in similar procedures.

Referring still to FIG. 16B, in an optional step 736 of the method the treatment target area is cleared via irrigation and/or suction. This optional step 736 can be used one or more times in the method and at various points in the method, such as between steps 711 and 721, between steps 721 and 731, between steps 731 and 741, between steps 741 and 751, and between steps 751 and 756.

Among the desirable features of the systems and methods disclosed herein is the ability to manipulate and stabilize tissue to create a fluid channel. Generally speaking, while the systems and methods provide the ability to manipulate and stabilize various and multiple locations related to the treatment of BPH, some locations within the anatomy may yield a greater therapeutic effect than others. These locations of greatest effect may vary from patient to patient or may be generally understood. The methods of the disclosure expressly involve identifying the desirability of a treatment target location based on a given patient's anatomy, disease state, and other relevant factors.

### Examples

The Detailed Description is intended to be illustrative and not restrictive. For example, the above-described examples (or one or more features or components thereof) can be used in combination with each other. System 100 and system 400 may be used in sequence to treat a subject, for instance. In additional embodiments, part or all of the distal section of system 100 and system 400 may be interchangeable, such that a single device or system may be reversibly coupled with distal section 160 and distal section 460, or one or more components thereof, such as cutter body 170 and delivery body 470, respectively. Various features or components have been or can thus be grouped together to streamline the disclosure. This should not be interpreted as intending that an unclaimed disclosed feature is essential to any claim. Rather, inventive subject matter can lie in less than all features of a particular disclosed embodiment. While particular elements, embodiments and applications of the present invention have been shown and described, it will be understood that the invention is not limited thereto since modifications can be made by those skilled in the art without departing from the scope of the present disclosure, particularly in light of the foregoing teachings.

### Closing Notes

Certain terms are used throughout this patent document to refer to particular features or components. As one skilled in the art appreciates, different people may refer to the same feature or component by different names. This patent document does not intend to distinguish between components or features that differ in name but not in function.

For the following defined terms, certain definitions shall be applied unless a different definition is given elsewhere in this patent document. The terms "a," "an," and "the" are used to include one or more than one, independent of any other instances or usages of "at least one" or "one or more." The term "or" is used to refer to a nonexclusive or, such that "A or B" includes "A but not B," "B but not A," and "A and B." All numeric values are assumed to be modified by the term "about," whether or not explicitly indicated. The term "about" generally refers to a range of numbers that one of skill in the art would consider equivalent to the recited value (e.g., having the same function or result). In many instances, the term "about" can include numbers that are rounded to the nearest significant figure. The recitation of numerical ranges by endpoints includes all numbers and sub-ranges within and bounding that range (e.g., 1 to 4 includes 1, 1.5, 1.75, 2, 2.3, 2.6, 2.9, etc. and 1 to 1.5, 1 to 2, 1 to 3, 2 to 3.5, 2 to 4, 3 to 4, etc.). The terms "patient" and "subject" are intended to include mammals, such as for human or veterinary applications.

The scope of the invention should be determined with reference to the appended claims. In the appended claims, the terms "including" and "in which" are used as the plain-English equivalents of the respective terms "comprising" and "wherein." Also, in the following claims, the terms "including" and "comprising" are open-ended; that is, a device, kit or method that includes features or components in addition to those listed after such a term in a claim are still deemed to fall within the scope of that claim. Moreover, in the following claims, the terms "first," "second" and "third," etc. are used merely as labels, and are not intended to impose numerical requirements on their objects.

The Abstract is provided to allow the reader to quickly ascertain the nature of the technical disclosure. It is submitted with the understanding that it will not be used to interpret or limit the scope or meaning of the claims.

The invention is defined by the following claims.

## Claims

1. A system (100) for treating target tissue in a prostatic urethra, comprising:
a handle body (110) having a cutter actuator (112) and an inflation port (116);
an elongate member (150) coupled to a distal end of the handle body;
a cutter body (170) and an expandable member (180) coupled to a distal end of the elongate member, wherein the expandable member is expandable via fluid supplied through the inflation port via the elongate member and the expandable member is configured to stabilize the cutter body against target tissue when the expandable member is expanded;
a cutter (172) configured to move from a first position within the cutter body to a second position protruding from a cutter slot (175) and to a third position again within the cutter body, wherein movement of the cutter is controlled via the cutter actuator; and
an injection port (118) on the handle body in fluid communication with the cutter slot via the elongate member, wherein the injection port is configured to supply a therapeutic substance through the cutter slot to the target tissue.

2. The system of claim 1, wherein the cutter body and the expandable member are coupled to the distal end of the elongate member via an articulated section (165).

3. The system of claim 2, wherein the articulated section is configured to curve at a pre-formed angle with respect to a long axis of the elongate member.

4. The system of any one of the preceding claims, wherein the expandable member is configured to stabilize the cutter body against the target tissue by expanding away from the cutter body.

5. The system of claim 4, wherein the expandable member engages with tissue when it is expanded.

6. The system of any one of the preceding claims, wherein the cutter slot is positioned along a long axis of the cutter body.

7. The system of any one of the preceding claims, wherein the cutter is configured to move in a distal-to-proximal direction.

8. The system of any one of the preceding claims, wherein the cutter is configured to move in a proximal-to-distal direction.

9. The system of any one of the preceding claims, further comprising at least one cutter recess (176, 178) in the cutter body, wherein the cutter is configured to be entirely within the cutter body when at least a portion of the cutter is positioned in the cutter recess.

10. The system of claim 9, wherein
the at least one cutter recess comprises two cutter recesses; and
the two cutter recesses comprise a distal cutter recess (176) and a proximal cutter recess (178).

11. The system of claim 10, further comprising a cutter track (177) positioned between the distal cutter recess and the proximal cutter recess, wherein the cutter track is configured to position a cutting edge (184) of the cutter beyond a tissue-facing surface of the cutter body.

12. The system of claim 11, wherein the cutter track is configured to vary a depth of cut into tissue as the cutter moves along the cutter track.

13. The system of any one of the preceding claims, further comprising a cutter wire coupled between the cutter actuator and the cutter.

14. The system of claim 13, wherein
the cutter wire is positioned within the elongate member; and
the cutter wire is position within a cutter wire lumen within the elongate member.

15. The system of claim 14, wherein
the cutter wire lumen is in fluid communication with the injection port; and
the cutter wire lumen is configured to transmit fluid from the injection port to the cutter slot.

## Patentansprüche

1. System (100) zum Behandeln von Zielgewebe in einer prostatischen Harnröhre, umfassend:
einen Griffkörper (110) mit einem Schneidaktuator (112) und einer Aufblasöffnung (116);
ein längliches Element (150), das mit einem distalen Element des Griffkörpers gekoppelt ist;
einen Schneidkörper (170) und ein expandierbares Element (180), das mit einem distalen Ende des länglichen Elements gekoppelt ist, wobei das expandierbare Element über Fluid, das durch die Aufblasöffnung über das längliche Element zugeführt wird, expandierbar ist und das expandierbare Element dazu ausgelegt ist, den Schneidkörper gegen Zielgewebe zu stabilisieren, wenn das expandierbare Element expandiert wird;
ein Schneidelement (172), das dazu ausgelegt sich, sich von einer ersten Position innerhalb des Schneidkörpers zu einer zweiten Position, die von einem Schneidschlitz (175) wegsteht, und zu einer dritten Position wieder innerhalb des Schneidkörpers zu bewegen; und
eine Injektionsöffnung (118) an dem Griffkörper in Fluidkommunikation mit dem Schneidschlitz über das längliche Element, wobei die Injektionsöffnung dazu ausgelegt ist, eine therapeutische Substanz durch den Schneidschlitz dem Zielgewebe zuzuführen.

2. System nach Anspruch 1, wobei der Schneidkörper und das expandierbare Element über einen Gelenksabschnitt (165) mit dem distalen Ende des länglichen Elements gekoppelt sind.

3. System nach Anspruch 2, wobei der Gelenksabschnitt dazu ausgelegt ist, sich in einem vorgeformten Winkel in Bezug auf eine Längsachse des länglichen Elements zu krümmen.

4. System nach einem der vorangegangenen Ansprüche, wobei das expandierbare Element dazu ausgelegt ist, den Schneidkörper gegen das Zielgewebe zu stabilisieren, indem es von dem Schneidkörper weg expandiert wird.

5. System nach Anspruch 4, wobei das expandierbare Element mit Gewebe in Eingriff gelangt, wenn es expandiert wird.

6. System nach einem der vorangegangenen Ansprüche, wobei der Schneidschlitz entlang einer Längsachse des Schneidkörpers angeordnet ist.

7. System nach einem der vorangegangenen Ansprüche, wobei das Schneidelement dazu ausgelegt ist, sich in eine Distal-zu-proximal-Richtung zu bewegen.

8. System nach einem der vorangegangenen Ansprüche, wobei das Schneidelement dazu ausgelegt ist, sich in eine Proximal-zu-distal-Richtung zu bewegen.

9. System nach einem der vorangegangenen Ansprüche, ferner umfassend zumindest eine Schneidaussparung (176, 178) in dem Schneidkörper, wobei das Schneidelement dazu ausgelegt ist, vollständig innerhalb des Schneidkörpers zu sein, wenn zumindest ein Abschnitt des Schneidelements in der Schneidaussparung angeordnet ist.

10. System nach Anspruch 9, wobei
die zumindest eine Schneidaussparung zwei Schneidaussparungen umfasst; und
die zwei Schneidaussparungen eine distale Schneidaussparung (176) und eine proximale Schneidaussparung (178) umfassen.

11. System nach Anspruch 10, ferner umfassend eine Schneidbahn (177), die zwischen der distalen Schneidaussparung und der proximalen Schneidaussparung angeordnet ist, wobei die Schneidbahn dazu ausgelegt ist, eine Schneidekante (184) des Schneidelements über einer dem Gewebe zugewandten Fläche des Schneidkörpers hinweg anzuordnen.

12. System nach Anspruch 11, wobei die Schneidspur dazu ausgelegt ist, eine Schnitttiefe in Gewebe zu variieren, während sich das Schneidelement entlang der Schneidbahn bewegt.

13. System nach einem der vorangegangenen Ansprüche, ferner umfassend einen Schneiddraht, der zwischen dem Schneidaktuator und dem Schneidelement gekoppelt ist.

14. System nach Anspruch 13, wobei
der Schneiddraht innerhalb des länglichen Elements angeordnet ist; und
der Schneiddraht innerhalb eines Schneiddrahtlumen innerhalb des länglichen Elements angeordnet ist.

15. System nach Anspruch 14, wobei
das Schneiddrahtlumen in Fluidkommunikation mit der Injektionsöffnung ist; und
das Schneiddrahtlumen dazu ausgelegt ist, Fluid von der Injektionsöffnung zu dem Schneidschlitz zu übertragen.

## Revendications

1. Système (100) pour traiter du tissu cible dans un urètre prostatique, comprenant :
un corps de poignée (110) présentant un actionneur de dispositif de coupe (112) et un orifice de gonflage (116) ;
un élément allongé (150) couplé à une extrémité distale du corps de poignée ;
un corps de dispositif de coupe (170) et un élément extensible (180) couplés à une extrémité distale de l'élément allongé, dans lequel l'élément extensible est extensible via un fluide fourni à travers l'orifice de gonflage via l'élément allongé et l'élément extensible est configuré pour stabiliser le corps de dispositif de coupe contre un tissu cible lorsque l'élément extensible est dilaté ;
un dispositif de coupe (172) configuré pour se déplacer d'une première position à l'intérieur du corps de dispositif de coupe à une deuxième position faisant saillie à partir d'une fente de dispositif de coupe (175) et à une troisième position à nouveau à l'intérieur du corps de dispositif de coupe, dans lequel le déplacement du dispositif de coupe est commandé via l'actionneur de dispositif de coupe ; et
un orifice d'injection (118) sur le corps de poignée en communication fluidique avec la fente de dispositif de coupe via l'élément allongé, dans lequel l'orifice d'injection est configuré pour fournir une substance thérapeutique à travers la fente de dispositif de coupe au tissu cible.

2. Système selon la revendication 1, dans lequel le corps de dispositif de coupe et l'élément extensible sont couplés à l'extrémité distale de l'élément allongé via une section articulée (165).

3. Système selon la revendication 2, dans lequel la section articulée est configurée pour se courber selon un angle préformé par rapport à un axe long de l'élément allongé.

4. Système selon l'une quelconque des revendications précédentes, dans lequel l'élément extensible est configuré pour stabiliser le corps de dispositif de coupe contre le tissu cible en étant dilaté à l'écart du corps de dispositif de coupe.

5. Système selon la revendication 4, dans lequel l'élément extensible vient en prise avec le tissu lorsqu'il est dilaté.

6. Système selon l'une quelconque des revendications précédentes, dans lequel la fente de dispositif de coupe est positionnée le long d'un axe longitudinal du corps de dispositif de coupe.

7. Système selon l'une quelconque des revendications précédentes, dans lequel le dispositif de coupe est configuré pour se déplacer dans une direction distale à proximale.

8. Système selon l'une quelconque des revendications précédentes, dans lequel le dispositif de coupe est configuré pour se déplacer dans une direction proximale à distale.

9. Système selon l'une quelconque des revendications précédentes, comprenant en outre au moins un évidement de dispositif de coupe (176, 178) dans le corps de dispositif de coupe, dans lequel le dispositif de coupe est configuré pour être entièrement à l'intérieur du corps de dispositif de coupe lorsqu'au moins une partie du dispositif de coupe est positionnée dans l'évidement de dispositif de coupe.

10. Système selon la revendication 9, dans lequel le au moins un évidement de dispositif de coupe comprend deux évidements de dispositif de coupe ; et
les deux évidements de dispositif de coupe comprennent un évidement de dispositif de coupe distal (176) et un évidement de dispositif de coupe proximal (178).

11. Système selon la revendication 10, comprenant en outre une piste de dispositif de coupe (177) positionnée entre l'évidement de dispositif de coupe distal et l'évidement de dispositif de coupe proximal, dans lequel la piste de dispositif de coupe est configurée pour positionner un bord de coupe (184) du dispositif de coupe au-delà d'une surface faisant face au tissu du corps de dispositif de coupe.

12. Système selon la revendication 11, dans lequel la piste de dispositif de coupe est configurée pour faire varier une profondeur de dispositif de coupe dans du tissu lorsque le dispositif de coupe se déplace le long de la piste de dispositif de coupe.

13. Système selon l'une quelconque des revendications précédentes, comprenant en outre un fil de dispositif de coupe couplé entre l'actionneur de dispositif de coupe et le dispositif de coupe.

14. Système selon la revendication 13, dans lequel :
le fil de dispositif de coupe est positionné à l'intérieur de l'élément allongé ; et
le fil de dispositif de coupe est positionné à l'intérieur d'une lumière de fil de dispositif de coupe à l'intérieur de l'élément allongé.

15. Système selon la revendication 14, dans lequel :
la lumière de fil de dispositif de coupe est en communication fluidique avec l'orifice d'injection ; et
la lumière de fil de dispositif de coupe est configurée pour transmettre le fluide de l'orifice d'injection à la fente de dispositif de coupe.
